# EUROPEAN PATENT APPLICATION

(11) **EP 4 467 638 A1**
(43) Date of publication of application: **27.11.2024**
(21) Application number: 23740286.2
(22) Date of filing: 12.01.2023
(51) Int. Cl.: C12N 5/071

(54) **METHOD FOR PRODUCING THREE-DIMENSIONAL CELL TISSUE**

(30) Priority: 17.01.2022 JP 2022005014
(71) Applicant: Toppan Holdings Inc., Tokyo 110-0016 (JP)
(72) Inventor: NADA Isana, Tokyo 110-0016 (JP); MORIMURA Rii, Tokyo 110-0016 (JP); KITANO Shiro, Tokyo 110-0016 (JP)
(74) Representative: Plasseraud IP
(86) International application number: PCT/JP2023/000554
(87) International publication number: WO 2023/136278

(57) **Abstract**

A method of producing three-dimensional cellular tissues includes the steps of: mixing stromal cells with a cationic substance, an extracellular matrix component and a polyelectrolyte to obtain a mixture; removing a liquid portion from the mixture to obtain a cell aggregate; culturing the cell aggregate in a medium to obtain a three-dimensional cellular tissue; and culturing the three-dimensional cellular tissue in a medium containing ascorbic acid and transforming growth factor-β (TGF-β), wherein a thickness of the three-dimensional cellular tissue remains greater than 50 µm for at least 3 days after culturing the three-dimensional cellular tissue in the medium containing ascorbic acid and TGF-β.

## Description

### [Technical Field]

The present invention relates to a method of producing three-dimensional cellular tissues.

The present application claims priority to Japanese Patent Application No. 2022-005014 filed January 17, 2022, the entire contents of which are incorporated herein by reference.

### [Background Art]

In recent years, the superiority of using three-dimensional cellular tissues organized three-dimensionally over cells grown on a flat plate has been shown in the fields of regenerative medicine, assay systems for drugs that require an environment close to that of a living body, and the like. Accordingly, various techniques for constructing three-dimensional cellular tissues in vitro have been developed.

PTL 1 discloses a method for producing three-dimensional cellular tissues, the method including the steps of: obtaining a mixture containing cells suspended in a solution containing at least a cationic buffer solution, an extracellular matrix component and a polyelectrolyte; collecting the cells from the obtained mixture to form cell aggregates on a substrate; and culturing the cells to obtain three-dimensional cellular tissues. Three-dimensional cellular tissues can be used for, for example, biological tissue models or solid tumor models for use in various assays such as drug screening.

### [Citation List]

### [Patent Literature]

PTL 1: JP 6639634 B

### [Summary of Invention]

### [Technical Problem]

However, the inventors of the present invention found that, when a three-dimensional cellular tissue is produced by the method described in PTL 1, the thickness of the three-dimensional cellular tissue may decrease over time and collagen in the three-dimensional cellular tissue may decrease. If the thickness of the three-dimensional cellular tissue decreases and the collagen in the three-dimensional cellular tissue also decreases, cancer cells disposed in the three-dimensional cellular tissue may be exposed due to the reduced thickness of the three-dimensional cellular tissue or the reproducibility of assessment of immune cell migration may be impaired, for example.

Therefore, the present invention has been made to provide a technique for producing three-dimensional cellular tissues that can suppress a decrease in thickness of a three-dimensional cellular tissue over time even when cultured over time, maintain the thickness of the three-dimensional cellular tissue, and suppress decrease in collagen in the three-dimensional cellular tissue.

### [Solution to Problems]

The present invention includes the following aspects.
[1] A method of producing three-dimensional cellular tissues including the steps of: mixing stromal cells with a cationic substance, an extracellular matrix component and a polyelectrolyte to obtain a mixture; removing a liquid portion from the mixture to obtain a cell aggregate; culturing the cell aggregate in a medium to obtain a three-dimensional cellular tissue; and culturing the three-dimensional cellular tissue in a medium containing ascorbic acid and transforming growth factor-β (TGF-β), wherein a thickness of the three-dimensional cellular tissue remains greater than 50 µm for at least 3 days after culturing the three-dimensional cellular tissue in the medium containing ascorbic acid and TGF-β.
[2] A method of producing three-dimensional cellular tissues comprising the steps of: mixing stromal cells with a cationic substance, an extracellular matrix component and a polyelectrolyte to obtain a first mixture; removing a liquid portion from the mixture to obtain a first cell aggregate; culturing the first cell aggregate in a medium to obtain a first three-dimensional cellular tissue; disposing target cells on the first three-dimensional cellular tissue; mixing stromal cells with a cationic substance, an extracellular matrix component and a polyelectrolyte to obtain a second mixture; removing a liquid portion from the second mixture to obtain a second cell aggregate; disposing the second cell aggregate to be in contact with the target cells; and culturing the second cell aggregate in a medium containing ascorbic acid and transforming growth factor-β (TGF-β) to obtain a second three-dimensional cellular tissue, wherein a total thickness of the first three-dimensional cellular tissue, the target cells and the second three-dimensional cellular tissue remains greater than 50 µm for at least 3 days after culturing the second three-dimensional cellular tissue in the medium containing ascorbic acid and TGF-β.
[3] The production method according to [2], wherein the target cells are cancer cells.
[4] The production method according to any one of [1] to [3], wherein the extracellular matrix component is selected from the group consisting of collagen, laminin, fibronectin, vitronectin, elastin, tenascin, entactin, fibrin, proteoglycan, and combinations thereof.
[5] The production method according to any one of [1] to [4], wherein the polyelectrolyte is selected from the group consisting of glycosaminoglycan, dextran sulfate, rhamnan sulfate, fucoidan, carrageenan, polystyrene sulfonic acid, polyacrylamide-2-methylpropanesulfonic acid, polyacrylic acid, and combinations thereof.

### [Advantageous Effects of Invention]

According to the present invention, a technique for producing three-dimensional cellular tissues can be provided that can suppress decrease in thickness of a three-dimensional cellular tissue even when cultured over time, maintain the thickness of the three-dimensional cellular tissue, and suppress decrease in collagen in the three-dimensional cellular tissue.

### [Brief Description of Drawings]

Fig. 1 is a representative micrograph of a three-dimensional cellular tissue observed in Experimental Example 1.
Fig. 2 is a representative micrograph of a thin slice of an HE-stained three-dimensional cellular tissue observed in Experimental Example 1.
Fig. 3 is a graph showing the results of measuring the thickness of the three-dimensional cellular tissue measured in Experimental Example 1.
Fig. 4 is a representative micrograph of a thin slice of a PSR-stained three-dimensional cellular tissue observed in Experimental Example 1.
Fig. 5 is a graph showing the results of measuring the PSR ratio measured in Experimental Example 1.
Fig. 6 is a representative micrograph of a thin slice of a PSR-stained three-dimensional cellular tissue observed in Experimental Example 2.
Fig. 7 is a graph showing the results of measuring the relative cell viability (relative viability) measured in Experimental Example 3.
Fig. 8 is a representative micrograph of a thin slice of an HE-stained three-dimensional cellular tissue observed in Experimental Example 4.
Fig. 9 is a graph showing the results of measuring the thickness of the three-dimensional cellular tissue measured in Experimental Example 4.

### [Description of Embodiments]

### (First Embodiment)

In one embodiment, the present invention provides a method of producing three-dimensional cellular tissues, including the steps of: mixing stromal cells with a cationic substance, an extracellular matrix component and a polyelectrolyte to obtain a mixture; removing a liquid portion from the mixture to obtain a cell aggregate; culturing the cell aggregate in a medium to obtain a three-dimensional cellular tissue; and culturing the three-dimensional cellular tissue in a medium containing ascorbic acid and transforming growth factor-β (hereinafter, also called TGF-β), wherein a thickness of the three-dimensional cellular tissue remains greater than 50 µm for at least 3 days after culturing the three-dimensional cellular tissue in the medium containing ascorbic acid and TGF-β.

According to the production method of the present embodiment, it is possible to suppress a decrease in thickness of the three-dimensional cellular tissue over time even when cultured over time, maintain the thickness of the three-dimensional cellular tissue, and suppress decrease in collagen. Specifically, the thickness of the three-dimensional cellular tissue can remain greater than 50 µm for at least 3 days after culturing in a medium containing ascorbic acid and TGF-β. In other words, the thickness of the three-dimensional cellular tissue can be maintained at greater than 50 µm for at least 3 days after the start of culturing in a medium containing ascorbic acid and TGF-β.

The thickness of a three-dimensional cellular tissue as described herein refers to the thickness of a slice obtained by cutting the three-dimensional cellular tissue along a line passing through the center of gravity in a direction perpendicular to the top of the three-dimensional cellular tissue, which is the thickness of a slice taken at substantially the center part of the three-dimensional cellular tissue. The shape of the three-dimensional cellular tissue depends on the vessel used to produce the three-dimensional cellular tissue, and may be a cylindrical shape, for example, when the three-dimensional cellular tissue is produced using a cylindrical cell culture insert. In this case, the shape of the three-dimensional cellular tissue as viewed from the upper side is circular, and the center of gravity as viewed from the upper side is the center of the circle. The shape of the three-dimensional cellular tissue is not limited to a cylindrical shape, and can be any shape according to the purpose. Specifically, for example, a polygonal prism shape such as a triangular prism shape or a rectangular prism shape can be exemplified.

In the production method of the present embodiment, the thickness of the three-dimensional cellular tissue for at least 3 days after culturing in a medium containing ascorbic acid and TGF-β is greater than 50 µm, and may be, for example, 55 µm or greater, preferably 60 µm or greater, more preferably 70 µm or greater, still more preferably 80 µm or greater, still more preferably 90 µm or greater, still more preferably greater than 100 µm, still more preferably 110 µm or greater, still more preferably 120 µm or greater, still more preferably 130 µm or greater, still more preferably 140 µm or greater, still more preferably 150 µm or greater, still more preferably 160 µm or greater, still more preferably 170 µm or greater, still more preferably 180 µm or greater, still more preferably 190 µm or greater, and still more preferably 200 µm or greater. The upper limit is not particularly limited, but may be, for example, 1,000 µm.

In addition, the culturing period during which the thickness of the three-dimensional cellular tissue remains greater than 50 µm is at least 3 days after culturing the three-dimensional cellular tissue in a medium containing ascorbic acid and TGF-β, and may be, for example, 4 days, preferably 5 days, more preferably 6 days, still more preferably 7 days, still more preferably 8 days, still more preferably 9 days, still more preferably 10 days, still more preferably 11 days, still more preferably 12 days, still more preferably 13 days, still more preferably 14 days, still more preferably 15 days, still more preferably 16 days, still more preferably 17 days, still more preferably 18 days, still more preferably 19 days, and still more preferably 20 days.

The form of the three-dimensional cellular tissue is not particularly limited, and may be, for example, a three-dimensional cellular tissue formed by culturing cells in a scaffold of a natural biopolymer such as collagen or a synthetic polymer, a cell aggregate (also referred to as a spheroid), or a sheet-like cellular structure.

The term "three-dimensional cellular tissue" as used herein refers to a three-dimensional cell aggregate. Examples of the uses of the three-dimensional cellular tissue include, but are not limited to, biological tissue models and solid tumor models. Examples of the biological tissue models include skin, hair, bone, cartilage, tooth, cornea, blood vessel, lymphatic vessel, heart, liver, pancreas, nerve and esophagus models. Examples of the solid tumor models include gastric cancer, esophageal cancer, colorectal cancer, colon cancer, rectal cancer, pancreatic cancer, breast cancer, ovarian cancer, prostate cancer, renal cell cancer and liver cancer models.

Further, the form of the three-dimensional cellular tissue is not particularly limited, and may be, for example, a three-dimensional cellular tissue formed by culturing cells in a vessel such as a cell culture insert, a three-dimensional cellular tissue formed by culturing cells in a scaffold of a natural biopolymer such as collagen or a synthetic polymer, a cell aggregate, or a sheet-like cellular structure.

The production method of the present embodiment includes the steps of: (A) mixing stromal cells with a cationic substance, an extracellular matrix component and a polyelectrolyte to obtain a mixture; (B) removing a liquid portion from the mixture to obtain a cell aggregate; (C) culturing the cell aggregate in a medium to obtain a three-dimensional cellular tissue; and (D) culturing the three-dimensional cellular tissue in a medium containing ascorbic acid and TGF-β. Each step will be described below.

In step (A), the population of cells includes at least stromal cells. The stromal cells refer to cells constituting supporting tissues for epithelial cells. Example of the stromal cells used in the present embodiment include fibroblasts, immune cells, vascular endothelial cells and smooth muscle cells. Examples of the immune cells include lymphocytes, neutrophils and macrophages. Stromal cells are essential for maintenance of tissues, and play important roles in inflammatory responses, wound healing responses, and the like. In the present invention, the stromal cells can maintain the target cells.

The origin of the stromal cells are not particularly limited, and cells derived from mammals such as humans, monkeys, dogs, cats, rabbits, pigs, cows, mice and rats can be used.

### (Cationic Substance)

As the cationic substance, any substance having a positive charge can be used as long as it does not adversely affect the growth of stromal cells and formation of stromal cell aggregate described later. Examples of the cationic substance include, but are not limited to, cationic buffers, such as tris-HCl, tris-maleate, bis-tris and HEPES, ethanolamine, diethanolamine, triethanolamine, polyvinylamine, polyallylamine, polylysine, polyhistidine, polyarginine, and the like. In particular, a cationic buffer is preferred, and tris-HCl is more preferred.

When a cationic buffer is used as the cationic substance, the pH of the cationic buffer solution is not particularly limited as long as it does not adversely affect the growth of stromal cells and formation of stromal cell aggregate. The pH of the cationic buffer solution used in the present embodiment is preferably 6.0 to 8.0. For example, the pH of the cationic buffer solution used in the present embodiment may be 7.0, 7.1, 7.2, 7.3, 7.4, 7.5, 7.6, 7.7, 7.8, 7.9 or 8.0. The pH of the cationic buffer solution used in the present embodiment is more preferably 7.2 to 7.6, and still more preferably approximately 7.4.

### (Extracellular Matrix Component)

As the extracellular matrix component, any component constituting an extracellular matrix (also called ECM) can be used as long as it does not adversely affect the growth of stromal cells and formation of stromal cell aggregate described later. Examples of the extracellular matrix component include, but are not limited to, collagen, laminin, fibronectin, vitronectin, elastin, tenascin, entactin, fibrin, proteoglycan, and combinations thereof. The extracellular matrix component may also be modified forms or variants of the above components. These extracellular matrix components may be used singly or in combination of two or more.

Examples of the proteoglycan include chondroitin sulfate proteoglycan, heparan sulfate proteoglycan, keratan sulfate proteoglycan and dermatan sulfate proteoglycan. In particular, collagen, laminin and fibronectin are preferred, and collagen is particularly preferred as the extracellular matrix component.

The total content of the extracellular matrix component in the mixture in step (A) is not particularly limited as long as it does not adversely affect the growth of stromal cells and formation of stromal cell aggregate, and may be 0.005 mg/mL or greater and 1.5 mg/mL or less, preferably 0.005 mg/mL or greater and 1.0 mg/mL or less, more preferably 0.01 mg/mL or greater and 1.0 mg/mL or less, still more preferably 0.025 mg/mL or greater and 1.0 mg/mL or less, and still more preferably 0.025 mg/mL or greater and 0.1 mg/mL or less. The extracellular matrix component may be dissolved in an appropriate solvent before use. Examples of the solvent include, but are not limited to, water, buffer solutions and acetic acid. In particular, the solvent is preferably a buffer solution or acetic acid.

### (Polyelectrolyte)

The term "polyelectrolyte" as used herein refers to a polymer having a dissociable functional group in the polymer chain. As the polyelectrolyte used in the present embodiment, any polyelectrolyte can be used as long as it does not adversely affect the growth of stromal cells and formation of stromal cell aggregate. Examples of the polyelectrolyte include, but are not limited to, glycosaminoglycans such as heparin, chondroitin sulfate (e.g., chondroitin 4-sulfate, or chondroitin 6-sulfate), heparan sulfate, dermatan sulfate, keratan sulfate and hyaluronic acid; dextran sulfate, rhamnan sulfate, fucoidan, carrageenan, polystyrene sulfonic acid, polyacrylamide-2-methylpropanesulfonic acid, polyacrylic acid, and combinations thereof. The polyelectrolyte may be derivatives of those described above. These polyelectrolytes may be used singly or in combination of two or more.

The polyelectrolyte is preferably a glycosaminoglycan. In particular, heparin, chondroitin sulfate and dermatan sulfate are preferred, and heparin is particularly preferred.

The concentration of the polyelectrolyte in the mixture in step (A) is not particularly limited as long as it does not adversely affect the growth of stromal cells and formation of stromal cell aggregate. Unlike the extracellular matrix component, the polyelectrolyte is effective at any concentration as long as it is below the solubility limit, and does not inhibit the effects of the extracellular matrix component. The concentration of polyelectrolyte is preferably 0.005 mg/mL or greater, more preferably 0.005 mg/mL or greater and 1.0 mg/mL or less, still more preferably 0.01 mg/mL or greater and 1.0 mg/mL or less, still more preferably 0.025 mg/mL or greater and 1.0 mg/mL or less, and still more preferably 0.025 mg/mL or greater and 0.1 mg/mL or less.

The polyelectrolyte may be dissolved in an appropriate solvent before use. Examples of the solvent include, but are not limited to, water and buffer solutions. When a cationic buffer solution is used as the above cationic substance, the polyelectrolyte may be dissolved in a cationic buffer solution before use.

A mixing ratio (final concentration ratio) between the polyelectrolyte and the extracellular matrix component in the mixture in step (A) is preferably 1:2 to 2:1, more preferably 1:1.5 to 1.5:1, and still more preferably 1:1.

In step (A), the stromal cells, the cationic substance, the extracellular matrix component and the polyelectrolyte can be mixed in a suitable vessel such as a dish, tube, flask, bottle, well plate or cell culture insert. The mixing may be performed in a vessel used in step (B).

Subsequently, in step (B), a liquid portion is removed from the mixture to obtain a cell aggregate. The term "cell aggregate" as used herein refers to a population of cells. The cell aggregate includes cell precipitates obtained by centrifugation, filtration, or the like. In an embodiment, the cell aggregate is in the form of a slurry-like viscous body. The term "slurry-like viscous body" refers to a gel-like cell aggregate as described in Akihiro Nishiguchi et al., Cell-cell crosslinking by bio-molecular recognition of heparin-based layer-by-layer nanofilms, Macromol Biosci., 15 (3), 312-317, 2015.

The liquid portion can be removed using a method known to those skilled in the art. For example, the liquid portion may be removed by centrifugation or filtration. The conditions of centrifugation are not particularly limited as long as they do not adversely affect the growth of cells and formation of cell aggregate. For example, the liquid portion may be removed by centrifuging a microtube containing the mixture at room temperature at 400 to 1,000 × g for 2 minutes to separate the liquid portion from the cell aggregate. Alternatively, the liquid portion may be removed after collecting the cells by spontaneous sedimentation. As a result, a cell aggregate can be obtained.

Subsequently, in step (C), the cell aggregate is cultured in a medium to obtain a three-dimensional cellular tissue. The cell aggregate may be suspended in a solution before culture. The solution is not particularly limited as long as it does not adversely affect the growth of cells and formation of three-dimensional cellular tissue. For example, a cell culture medium, a buffer solution, or the like suitable for the cells constituting the cell aggregate can be used. The suspension of the cell aggregate can be performed in a suitable vessel such as a dish, tube, flask, bottle or plate.

When the cell aggregate is suspended in a solution, the cells may be precipitated before culture to form a cell precipitate on a substrate. The substrate may be a culture vessel used for cell culture. The culture vessel may be a vessel having a material and a shape typically used for cell or microorganism culture. Examples of the material of the culture vessel include, but are not limited to, glass, stainless steel and plastic. Examples of the culture vessel include, but are not limited to, a dish, tube, flask, bottle and plate. The substrate may be, for example, a material that allows liquid to pass through without allowing cells in the liquid to pass through. The substrate is preferably a permeable membrane. Examples of vessels having a permeable membrane include, but are not limited to, cell culture inserts such as Transwell (registered trademark) insert, Netwell (registered trademark) insert, Falcon (registered trademark) cell culture insert, and Millicell (registered trademark) cell culture insert.

Precipitation of cells can be performed, for example, by centrifugation. The conditions of centrifugation are not particularly limited as long as they do not adversely affect the growth of cells and formation of cell aggregate. For example, a suspension of a cell aggregate may be subjected to centrifugation at room temperature at 400 to 1,000 × g for 2 minutes to cause precipitation of cells. Alternatively, the cells may be precipitated by spontaneous sedimentation.

A method known to those skilled in the art can be used to precipitate the cells. For example, the cells may be collected by centrifugation, magnetic separation or filtration. The conditions of centrifugation are not particularly limited as long as they do not adversely affect the growth of cells. For example, the cells may be collected by seeding the mixture or suspension in a cell culture insert and centrifuging it at room temperature at 400 × g for 2 minutes. Alternatively, the cells may be collected by spontaneous sedimentation. Further, the collected cells may form a layer structure.

The cell aggregate, or the suspended cells when the cell aggregate is suspended, may be seeded at a cell density of, for example, 1,000 cells/mm² or greater, preferably 10,000 cells/mm² or greater, more preferably 20,000 cells/mm² or greater, and still more preferably 25,000 cells/mm² or greater. Further, the cells can be seeded at a cell density of, for example, 1,000,000 cells/mm² or less, preferably 500,000 cells/mm² or less, more preferably 200,000 cells/mm² or less, and still more preferably 100,000/mm² or less. By performing the steps of suspending the cell aggregate in a solution and precipitating the cells, a more homogeneous three-dimensional cellular tissue can be obtained. The upper and lower limits of the cell density can be combined as appropriate.

In step (C), the cells can be cultured under culture conditions suitable for the cells to be cultured. Those skilled in the art can select a suitable medium depending on the cell type and desired function. The cell culture medium is not particularly limited, but may be a basic medium such as DMEM, E-MEM, MEMα, RPMI-1640, Mccoy'5a or Ham's F-12, or a medium obtained by adding CS (also called bovine serum), FBS (also called fetal bovine serum) or HBS (also called fetal horse serum) to a basic medium so that the serum content is about 1% to 20% by volume. Conditions such as the temperature and the atmospheric composition of the culture environment can also be easily determined by those skilled in the art.

During cell culture, a substance for suppressing deformation of the constructed three-dimensional cellular tissue (such as shrinkage of tissue, peeling at the tissue edge, or the like) may be added to the medium. Examples of such a substance include, but are not limited to, Y-27632, which is a selective ROCK (also called Rho-associated coiled-coil forming kinase/Rho binding kinase) inhibitor.

Subsequently, in step (D), the three-dimensional cellular tissue obtained in step (C) is cultured in a medium containing ascorbic acid and TGF-β. Examples of the medium containing ascorbic acid and TGF-β include a medium obtained by adding ascorbic acid and TGF-β to the cell culture medium used in step (C).

### (Ascorbic Acid)

Ascorbic acid is an organic compound having a lactone structure, and is also known as vitamin C, which is a water-soluble vitamin. Ascorbic acid is an optically active compound and exists in L- and D-forms, with the L-form being preferred. The L-form is known as vitamin C. The ascorbic acid may be an ascorbate or an ascorbic acid derivative. Examples of the ascorbate include sodium ascorbate, potassium ascorbate and calcium ascorbate. Examples of the ascorbic acid derivative include magnesium ascorbate phosphate, sodium ascorbate phosphate and ascorbic acid 2-glucoside.

The concentration of the ascorbic acid in the medium is not particularly limited as long as the thickness of the three-dimensional cellular tissue can remain greater than 50 µm for at least 3 days after culturing in the medium containing ascorbic acid and TGF-β, and may be 0.02 mM or greater and 0.7 mM or less, preferably 0.02 mM or greater and 0.5 mM or less, more preferably 0.02 mM or greater and 0.3 mM or less, and still more preferably 0.02 mM or greater and 0.1 mM or less, or may be 0.05 mM or greater and 0.7 mM or less, preferably 0.05 mM or greater and 0.5 mM or less, more preferably 0.05 mM or greater and 0.3 mM or less, and still more preferably 0.05 mM or greater and 0.1 mM or less, or may be 0.07 mM or greater and 0.7 mM or less, preferably 0.07 mM or greater and 0.5 mM or less, more preferably 0.07 mM or greater and 0.3 mM or less, and still more preferably 0.07 mM or greater and 0.1 mg/mL or less.

### (TGF-β)

TGF-β (transforming growth factor-β) is a cytokine belonging to TGF-β family and exists in three isoforms in mammals (specifically, TGF-β1, TGF-β2 and TGF-β3). TGF-β plays an important role in cell proliferation, cell death, cell differentiation, immune regulation and cell motility, and the like. In the production method of the present embodiment, the TGF-β may be any of TGF-β1, TGF-β2 and TGF-β3.

The concentration of the TGF-β in the medium is not particularly limited as long as the thickness of three-dimensional cellular tissue can remain greater than 50 µm for at least 3 days after culturing in the medium containing ascorbic acid and TGF-β, and may be 0.05 ng/mL or greater and 15 ng/mL or less, preferably 0.05 ng/mL or greater and 12 ng/mL or less, more preferably 0.05 ng/mL or greater and 10 ng/mL or less, still more preferably 0.05 ng/mL or greater and 7 ng/mL or less, and still more preferably 0.05 ng/mL or greater and 5 ng/mL or less, or may be 0.07 ng/mL or greater and 15 ng/mL or less, preferably 0.07 ng/mL or greater and 12 ng/mL or less, more preferably 0.07 ng/mL or greater and 10 ng/mL or less, still more preferably 0.07 ng/mL or greater and 7 ng/mL or less, and still more preferably 0.07 ng/mL or greater and 5 ng/mL or less, or may be 0.1 ng/mL or greater and 15 ng/mL or less, preferably 0.1 ng/mL or greater and 12 ng/mL or less, more preferably 0.1 ng/mL or greater and 10 ng/mL or less, still more preferably 0.1 ng/mL or greater and 7 ng/mL or less, and still more preferably 0.1 ng/mL or greater and 5 ng/mL or less, or may be 0.2 ng/mL or greater and 15 ng/mL or less, preferably 0.2 ng/mL or greater and 12 ng/mL or less, more preferably 0.2 ng/mL or greater and 10 ng/mL or less, still more preferably 0.2 ng/mL or greater and 7 ng/mL or less, and still more preferably 0.2 ng/mL or greater and 5 ng/mL or less, or may be 0.5 ng/mL or greater and 15 ng/mL or less, preferably 0.5 ng/mL or greater and 12 ng/mL or less, more preferably 0.5 ng/mL or greater and 10 ng/mL or less, still more preferably 0.5 ng/mL or greater and 7 ng/mL or less, and still more preferably 0.5 ng/mL or greater and 5 ng/mL or less.

### (Second Embodiment)

In one embodiment, the present invention provides a method of producing three-dimensional cellular tissues including the steps of: mixing stromal cells with a cationic substance, an extracellular matrix component and a polyelectrolyte to obtain a first mixture; removing a liquid portion from the mixture to obtain a first cell aggregate; culturing the first cell aggregate in a medium to obtain a first three-dimensional cellular tissue; disposing target cells on the first three-dimensional cellular tissue; mixing stromal cells with a cationic substance, an extracellular matrix component and a polyelectrolyte to obtain a second mixture; removing a liquid portion from the second mixture to obtain a second cell aggregate; disposing the second cell aggregate to be in contact with the target cells; and culturing the second cell aggregate in a medium containing ascorbic acid and transforming growth factor-β (also called TGF-β) to obtain a second three-dimensional cellular tissue, wherein a total thickness of the first three-dimensional cellular tissue, the target cells and the second three-dimensional cellular tissue remains greater than 40 µm for at least 3 days after culturing the second three-dimensional cellular tissue in the medium containing ascorbic acid and TGF-β.

According to the production method of the present embodiment, it is possible to suppress a decrease in thickness of the three-dimensional cellular tissue over time even when cultured over time, maintain the thickness of the three-dimensional cellular tissue, and suppress decrease in collagen in the three-dimensional cellular tissue. Specifically, as will be described later in the examples, the thickness of the three-dimensional cellular tissue can remain greater than 50 µm for at least 3 days after culturing in a medium containing ascorbic acid and TGF-β. In the present embodiment, the thickness of the three-dimensional cellular tissue means the total thickness of the first three-dimensional cellular tissue, the target cells and the second three-dimensional cellular tissue.

In the production method of the present embodiment, the thickness of the three-dimensional cellular tissue for at least 3 days after culturing in a medium containing ascorbic acid and TGF-β is greater than 50 µm, and may be, for example, 55 µm or greater, preferably 60 µm or greater, more preferably 70 µm or greater, still more preferably 80 µm or greater, still more preferably 90 µm or greater, still more preferably greater than 100 µm, still more preferably 110 µm or greater, still more preferably 120 µm or greater, still more preferably 130 µm or greater, still more preferably 140 µm or greater, still more preferably 150 µm or greater, still more preferably 160 µm or greater, still more preferably 170 µm or greater, still more preferably 180 µm or greater, still more preferably 190 µm or greater, and still more preferably 200 µm or greater. The upper limit is not particularly limited, but may be, for example, 1,000 µm.

In addition, the culturing period during which the thickness of the three-dimensional cellular tissue remains greater than 50 µm is at least 3 days after culturing the three-dimensional cellular tissue in a medium containing ascorbic acid and TGF-β, and may be, for example, 4 days, preferably 5 days, more preferably 6 days, still more preferably 7 days, still more preferably 8 days, still more preferably 9 days, still more preferably 10 days, still more preferably 11 days, still more preferably 12 days, still more preferably 13 days, still more preferably 14 days, still more preferably 15 days, still more preferably 16 days, still more preferably 18 days, still more preferably 19 days, and still more preferably 20 days.

In the production method of the present embodiment, the thickness of the three-dimensional cellular tissue, the meaning of three-dimensional cellular tissue, and the form of the three-dimensional cellular tissue are as described in the first embodiment.

The production method of the present embodiment includes the steps of: (A1) mixing stromal cells with a cationic substance, an extracellular matrix component and a polyelectrolyte to obtain a first mixture; (B1) removing a liquid portion from the mixture to obtain a first cell aggregate; culturing the first cell aggregate in a medium to obtain a first three-dimensional cellular tissue; (C1) disposing the target cells on the first three-dimensional cellular tissue; (A2) mixing stromal cells with a cationic substance, an extracellular matrix component and a polyelectrolyte to obtain a second mixture; (B2) removing a liquid portion from the second mixture to obtain a second cell aggregate; (C2) disposing the second cell aggregate to be in contact with the target cells; and (D1) culturing the second cell aggregate in a medium containing ascorbic acid and TGF-β. Each step will be described below.

In the present embodiment, step (A1) and step (B1) are the same as step (A) and step (B) in the first embodiment, respectively.

Next, in step (C1), a population of target cells is disposed on the first three-dimensional cellular tissue obtained in step (B 1). The term "target cells" refers to cells whose maintenance is to be controlled using stromal cells. The target cells are not particularly limited, and cells derived from mammals such as humans, monkeys, dogs, cats, rabbits, pigs, cows, mice and rats can be used. Also, the site of origin of the target cells is not particularly limited, and the target cells may be somatic cells, such as those derived from bone, muscle, viscera, nerve, brain, skin and blood, or may be reproductive cells, or cancer cells.

Examples of the somatic cells derived from blood include immune cells such as lymphocytes, neutrophils, macrophages and dendritic cells. Examples of the cancer cells include cells of gastric cancer, esophageal cancer, colorectal cancer, colon cancer, rectal cancer, pancreatic cancer, breast cancer, ovarian cancer, prostate cancer, renal cell cancer and liver cancer.

Moreover, the target cells may be pluripotent stem cells such as induced pluripotent stem cells (also called iPS cells) and embryonic stem cells (also called ES cells), or may be tissue stem cells.

The target cells may be primary cells or cultured cells such as subcultured cells and cell line cells. These target cells may be used singly or in combination of two or more.

After step (C1), in step (C2), the second cell aggregate, which is obtained by performing step (A2) of mixing stromal cells with a cationic substance, an extracellular matrix component and a polyelectrolyte to obtain a second mixture and step (B2) of removing a liquid portion from the second mixture to obtain a second cell aggregate, is disposed in contact with the target cells, which have been disposed on the first three-dimensional cellular tissue in step (C1). Step (A2) and step (B2) are the same as step (A) and step (B) in the first embodiment, respectively.

Subsequently, in step (D1), the second cell aggregate is cultured in a medium containing ascorbic acid and TGF-β. The method of culturing the second cell aggregate in a medium containing ascorbic acid and TGF-β is the same as the method described in the first embodiment.

It can be said that the production method of the present embodiment is a method of maintaining the thickness of a three-dimensional cellular tissue. Further, in the production method of the present embodiment, ascorbic acid and TGF-β can be said to be thickness-maintaining agents for maintaining the thickness of the three-dimensional cellular tissue. Further, ascorbic acid and TGF-β can be said to be improvement agents for suppressing a decrease in thickness of the three-dimensional cellular tissue.

The present invention includes other aspects as follows.
[1] A method of producing three-dimensional cellular tissues including the steps of: mixing stromal cells with a cationic substance, an extracellular matrix component and a polyelectrolyte to obtain a mixture; removing a liquid portion from the mixture to obtain a cell aggregate; culturing the cell aggregate in a medium to obtain a three-dimensional cellular tissue; and culturing the three-dimensional cellular tissue in a medium containing ascorbic acid and transforming growth factor-β (TGF-β), wherein the medium containing ascorbic acid and transforming growth factor-β (TGF-β) contains 0.02 mM or greater and 0.3 mM or less ascorbic acid and 0.05 ng/mL or greater and 12 ng/mL or less transforming growth factor-β, and the thickness of the three-dimensional cellular tissue is maintained at greater than 50 µm for at least 3 days after the start of culturing the three-dimensional cellular tissue in the medium containing ascorbic acid and TGF-β.
[2] A method of producing three-dimensional cellular tissues including the steps of: mixing stromal cells with a cationic substance, an extracellular matrix component and a polyelectrolyte to obtain a first mixture; removing a liquid portion from the mixture to obtain a first cell aggregate; culturing the first cell aggregate in a medium to obtain a first three-dimensional cellular tissue; disposing target cells on the first three-dimensional cellular tissue; mixing stromal cells with a cationic substance, an extracellular matrix component and a polyelectrolyte to obtain a second mixture; removing a liquid portion from the second mixture to obtain a second cell aggregate; disposing the second cell aggregate to be in contact with the target cells; and culturing the second cell aggregate in a medium containing ascorbic acid and transforming growth factor-β (TGF-β) to obtain a second three-dimensional cellular tissue, wherein the medium containing ascorbic acid and transforming growth factor-β (TGF-β) contains 0.02 mM or greater and 3 mM or less ascorbic acid and 0.05 ng/mL or greater and 12 ng/mL or less transforming growth factor-β, and a total thickness of the first three-dimensional cellular tissue, the target cells and the second three-dimensional cellular tissue is maintained at greater than 50 µm for at least 3 days after the start of culturing the second three-dimensional cellular tissue in the medium containing ascorbic acid and TGF-β.
[3] The production method according to [2], wherein the target cells are cancer cells.
[4] The production method according to any one of [1] to [3], wherein the extracellular matrix component is collagen.
[5] The production method according to any one of [1] to [4], wherein the polyelectrolyte is glycosaminoglycan.
[6] The production method according to any one of [1] to [5], wherein the medium containing ascorbic acid and transforming growth factor-β (TGF-β) contains 0.07 mM or greater and 0.1 mM or less ascorbic acid and 0.07 ng/mL or greater and 12 ng/mL or less transforming growth factor-β.

### Examples

The present invention will be more specifically described in greater detail below by way of examples. However, the following examples should not limit the scope of the present invention.

### [Experimental Example 1]

### (Production 1 of Three-Dimensional Cellular Tissues)

### <<Preparation of Stromal Cells>>

A DMEM medium (product number "043-30085", FUJIFII,M Wako Pure Chemical Corporation) containing 10% fetal bovine serum (FBS) and 1% antibiotic solution (penicillin, streptomycin) was prepared (hereinafter, also referred to as a "general-purpose medium").

1.0×10⁶ cells of human neonatal dermal fibroblasts NHDF (product number "CC-2509", Lonza) and 1.5×10⁴ cells of human umbilical vein endothelial cells HUVEC (product number "C2517A", Lonza) were suspended in a 50 mM tris-HCl buffer solution (pH 7.4) containing 0.1 mg/mL heparin (product number "H3149-100KU", Sigma) and 0.1 mg/mL collagen (product number "ASC-1-100-100", Sigma).

Then, the cell suspension was centrifuged at room temperature at 1,000 × g (gravitational acceleration) for 2 minutes, and, after removing the supernatant, resuspended in 300 µL of general-purpose medium. Then, the obtained cell suspension was seeded inside a 24-well cell culture insert (product number "3470", Corning Incorporated) coated with 0.1 mg/mL fibronectin in advance, with 1 mL of general-purpose medium added to the outside.

Then, the cell culture insert was centrifuged at room temperature at 400 × g for 2 minutes, and then allowed to stand in a CO₂incubator (37°C, 5% CO₂) for 2 hours. Then, 1 mL of general-purpose medium was added to the outside the cell culture insert, and the cells were cultured in a CO₂ incubator (37°C, 5% CO₂) for 24 hours (the time at which this culture was started is referred to as the culture start time).

### <<Seeding of Cancer Cells>>

The medium outside the cell culture insert was removed, and 1 mL of general-purpose medium was added. Then, the medium inside the culture insert was removed, and 100 µL of a suspension obtained by suspending 1×10³ human colon adenocarcinoma cells HCT15/β2m in 100 µL of general-purpose medium was seeded in the above cell culture insert, and the cell culture insert was allowed to stand in a CO₂ incubator (37°C, 5% CO₂) for 2 hours.

### <<Lamination of Stromal Cells>>

1×10⁶ NHDF cells and 1.5×10⁴ HUVEC cells were suspended in a 50 mM tris-HCl buffer solution (pH 7.4) containing 0.1 mg/mL heparin (product number "H3149-100KU", Sigma) and 0.1 mg/mL collagen (product number "ASC-1-100-100", Sigma).

Then, the obtained cell suspension was centrifuged at room temperature at 1,000 × g for 2 minutes, and, after removing the supernatant, resuspended in 200 µL of general-purpose medium. Then, 200 µL of the general-purpose medium in which the cells were suspended was laminated on the cell culture insert in which the cancer cells had been seeded.

Then, the obtained cell culture insert was allowed to stand in a CO₂ incubator (37°C, 5% CO₂) for 2 hours.

### <<Addition of Ascorbic Acid and TGF-β>>

The medium inside and outside the above cell culture insert was removed, and 2 mL of general-purpose medium was added, using magnesium ascorbate phosphate (product number "013-12061", FUJIFELM Wako Pure Chemical Corporation) and TGF-β (product number "209-16544", FUJIFELM Wako Pure Chemical Corporation), with the ascorbic acid concentration being 0 mM, 0.05 mM or 0.1 mM and the TGF-β concentration being 0 ng/mL, 0.1 ng/mL, 1 ng/mL, 5 ng/mL or 10 ng/mL, and the cells were cultured in a CO₂ incubator (37°C, 5% CO₂) for 3 days. Then, the medium inside and outside the culture insert was removed, and 2.3 mL of general-purpose medium was added with the ascorbic acid concentration being 0 mM, 0.05 mM or 0.1 mM and the TGF-β concentration being 0 ng/mL, 0.1 ng/mL, 1 ng/mL, 5 ng/mL or 10 ng/mL, and the cells were cultured in a CO₂ incubator (37°C, 5% CO₂) for 3 days.

### <<Fixation of Three-Dimensional Cellular Tissues>>

On day 4 and day 7 of the culture, the obtained three-dimensional cellular tissue was washed twice with PBS, and 300 mL of 10% formalin buffer solution (product number "062-01661", FUJIFELM Wako Pure Chemical Corporation) was added to the cell culture insert, which was then allowed to stand for 15 minutes or more, and washed three times with PBS to fix the three-dimensional cellular tissue.

### <<Observation of Three-Dimensional Cellular Tissues>>

The obtained three-dimensional cellular tissue was observed in a bright field mode using a microscope system (Operetta CLS, PerkinElmer) to include the entire inside of the cell culture insert.

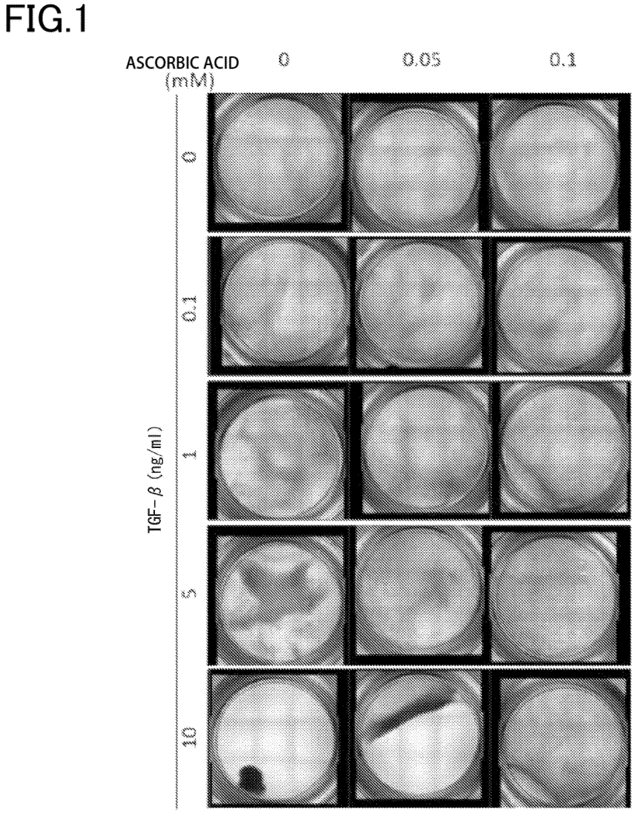

Fig. 1 is a photograph showing the results of microscopic observation of the three-dimensional cellular tissue on day 7 of culture. As shown in Fig. 1, when cultured in a medium containing ascorbic acid, a three-dimensional cellular tissue was formed. On the other hand, when cultured in a medium containing only TGF-β without containing ascorbic acid, the cellular tissue shrunk and became detached from the cell culture insert, and no three-dimensional cellular tissue was formed.

### <<Measurement of Thickness of Three-Dimensional Cellular Tissues>>

Then, the three-dimensional cellular tissue was removed from the cell culture insert, embedded in paraffin, and cut along a line passing through the center of gravity in a direction perpendicular to the top of the three-dimensional cellular tissue (top of the cell culture insert) to prepare a 6-mm thin slice. Then, the thin slice was stained with hematoxylin-eosin (HE), and the HE-stained thin slice was imaged using an optical microscope (MX51, Olympus Corporation) to measure a maximum thickness of the three-dimensional cellular tissue using ImageJ.

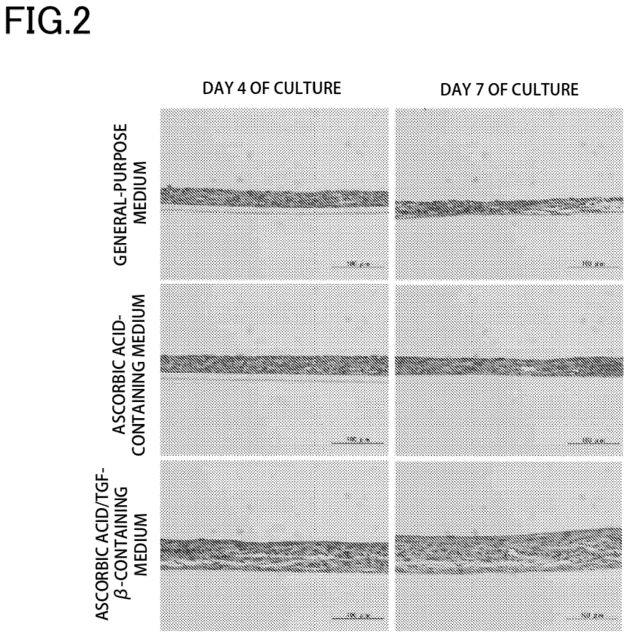

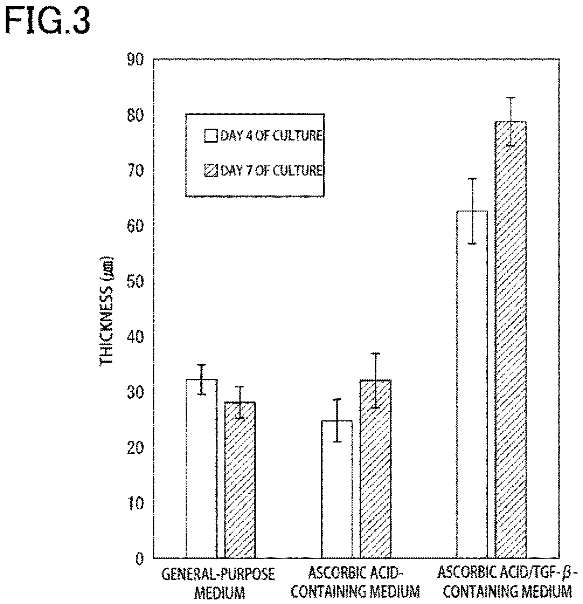

Fig. 2 shows the result of microscopic observation of the HE-stained three-dimensional cellular tissue, and Fig. 3 shows the result of measuring the thickness of the three-dimensional cellular tissue. In Figs. 2 and 3, "general-purpose medium" indicates the three-dimensional cellular tissue cultured in a general-purpose medium without containing ascorbic acid and TGF-β, "ascorbic acid-containing medium" indicates the three-dimensional cellular tissue cultured in a general-purpose medium with the ascorbic acid concentration of 0.1 mM, and "ascorbic acid/TGF-β-containing medium" indicates the three-dimensional cellular tissue cultured in a general-purpose medium with the ascorbic acid concentration of 0.1 mM and the TGF-β concentration of 10 ng/L. As shown in Figs. 2 and 3, the thickness of the three-dimensional cellular tissue was maintained by adding ascorbic acid and TGF-β, and the thickness of the three-dimensional cellular tissue was found to be greater than 50 µm even on day 4 of culture (on day 3 after addition of ascorbic acid and TGF-β).

### <<Formation of Collagen>>

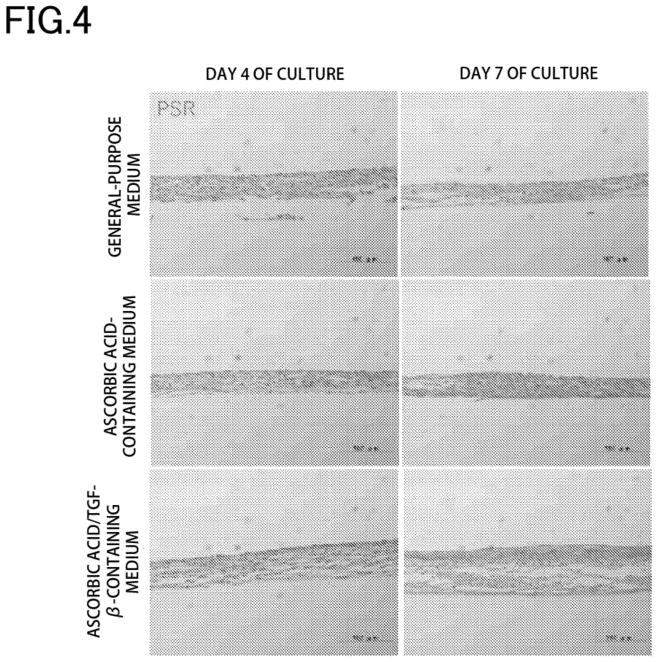

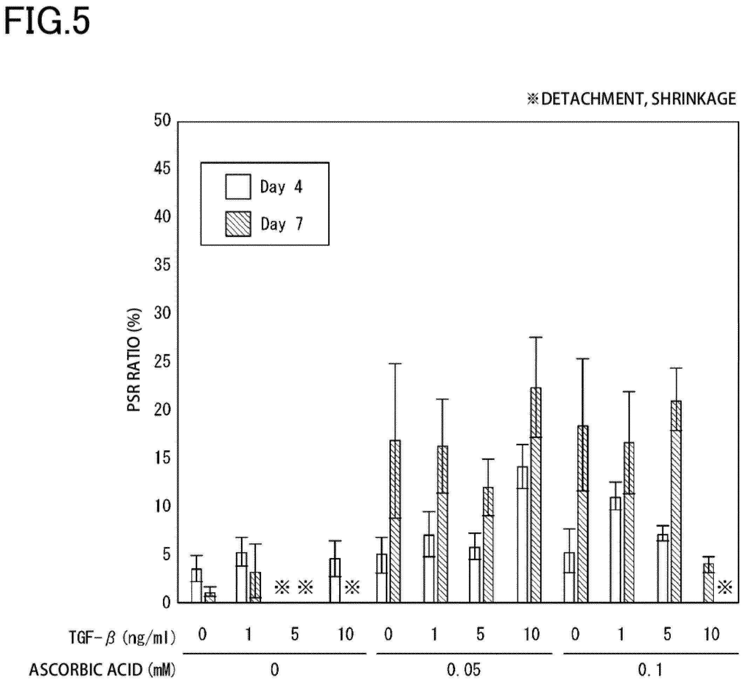

Then, the three-dimensional cellular tissue was removed from the cell culture insert, embedded in paraffin, and cut along a line passing through the center of gravity in a direction perpendicular to the top of the three-dimensional cellular tissue (top of the cell culture insert) to prepare a 6-mm thin slice. The obtained thin slice was stained with 0.1% Sirius Red-saturated picric acid (Pico-Sirius Red; PSR), and observed using an optical microscope (MX51, Olympus Corporation) to calculate the ratio of the PSR-stained area to the tissue area (PSR ratio). Fig. 4 shows the results of microscopic observation, and Fig. 5 shows the results of calculating the PSR ratio. In Fig. 4, "general-purpose medium" indicates the three-dimensional cellular tissue cultured in a general-purpose medium without containing ascorbic acid and TGF-β, "ascorbic acid-containing medium" indicates the three-dimensional cellular tissue cultured in a general-purpose medium with the ascorbic acid concentration of 0.1 mM, and "ascorbic acid/TGF-β-containing medium" indicates the three-dimensional cellular tissue cultured in a general-purpose medium with the ascorbic acid concentration of 0.1 mM and the TGF-β concentration of 1 ng/L.

As shown in Figs. 4 and 5, fiber-like collagen was formed by adding ascorbic acid, or by adding ascorbic acid and TGF-β.

### [Experimental Example 2]

### (Production 2 of Three-Dimensional Cellular Tissues)

Preparation of stromal cells, seeding of cancer cells and lamination of stromal cells were performed in the same manner as in Experimental Example 1 except that 2.0×10⁶ cells of human neonatal dermal fibroblasts NHDF were seeded, as NHDF, in a square dish (product number "166508", Nunc) containing 90 mL of general-purpose medium or general-purpose medium containing 0.1 mM ascorbic acid, and cultured in a CO₂ incubator (37°C, 5%CO₂) for 24 hours.

Then, the medium inside and outside the cell culture insert was removed, and 2.3 mL of general-purpose medium or general-purpose medium containing 0.1 mM ascorbic acid was added, and the cells were cultured in a CO₂ incubator (37°C, 5% CO₂) for 3 days. Subsequently, the medium inside and outside the culture insert was removed, and 2.3 mL of general-purpose medium or general-purpose medium containing 0.1 mM ascorbic acid was added, and the cells were cultured in a CO₂ incubator (37°C, 5% CO₂) for 3 days.

### <<Fixation of Three-Dimensional Cellular Tissues>>

On day 7 of the culture, the three-dimensional cellular tissue was washed twice with PBS, and 300 µL of 10% formalin buffer solution (product number "062-01661", FUJIFELM Wako Pure Chemical Corporation) was added to the cell culture insert, which was then allowed to stand for 15 minutes or more, and washed three times with PBS to fix the three-dimensional cellular tissue.

### <<Formation of Collagen>>

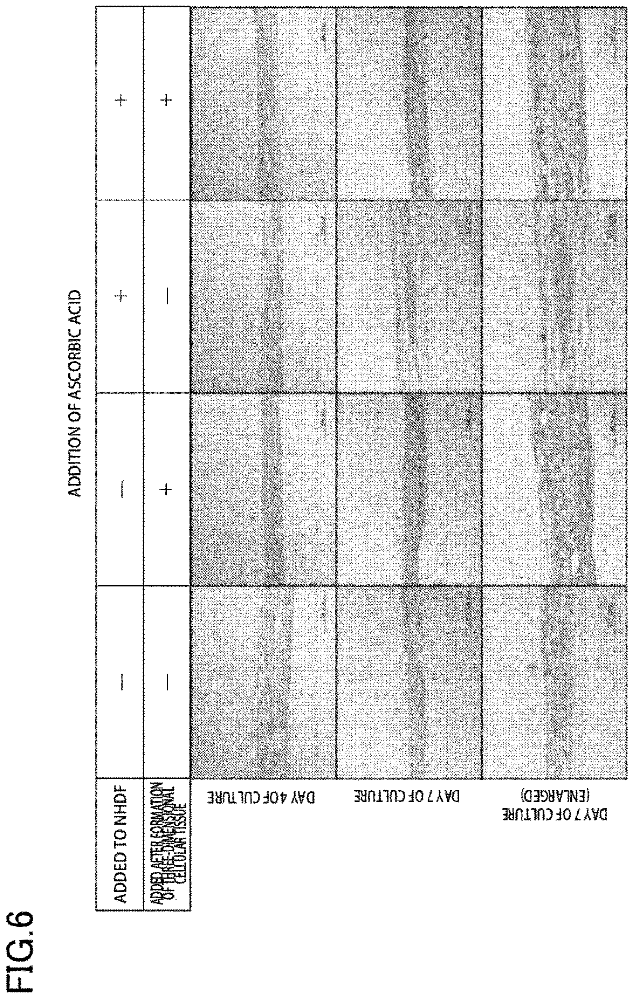

Then, the three-dimensional cellular tissue was removed from the cell culture insert, embedded in paraffin, and cut along a line passing through the center of gravity in a direction perpendicular to the top of the three-dimensional cellular tissue (top of the cell culture insert) to prepare a 6-mm thin slice. The obtained thin slice was stained with 0.1% Sirius Red-saturated picric acid (Pico-Sirius Red; PSR), and observed using an optical microscope (MX51, Olympus Corporation). Fig. 6 shows the results of microscopic observation.

As shown in Fig. 6, collagen was found to be formed when ascorbic acid was added after formation of the three-dimensional cellular tissue, but there was no change in formation of collagen in the three-dimensional cellular tissue even when ascorbic acid was added to NHDF in advance. This shows that culturing the three-dimensional cellular tissue in a medium containing ascorbic acid after formation of the three-dimensional cellular tissue is important for formation of collagen and maintaining the thickness of the three-dimensional cellular tissue.

### [Experimental Example 3]

### (Functional Evaluation of Three-Dimensional Cellular Tissues)

A three-dimensional cellular tissue was prepared in the same manner as in Experimental Example 1 except that a general-purpose medium containing 0.1 mM ascorbic acid and 1 ng/mL TGF-β was used as the general-purpose medium containing ascorbic acid and TGF-β, and GFP-expressing HCT15/β2m cells were used as the cancer cells.

An AIM-V medium (product number "A3830801", Gibco) containing 1% HEPES, 55 mM 2-mercaptoethanol and 1% antibiotic solution (penicillin, streptomycin) was prepared (hereinafter, also referred to as "medium for CTL").

On day 8 of culture, the medium outside the cell culture insert was removed, and 1 mL of medium for CTL was added. Then, the medium inside the cell culture insert was removed, and a CTL cell suspension obtained by suspending 4.0×10⁴ cells of cytotoxic T lymphocytes (also referred to as "CTL cells") in 300 µL of medium for CTL was added, and the cells were cultured in a CO₂ incubator (37°C, 5% CO₂) for 3 days. Also, cells cultured in the same manner without the addition of CTL cell suspension were used as controls.

### «Functional Evaluation of Three-Dimensional Cellular Tissues»

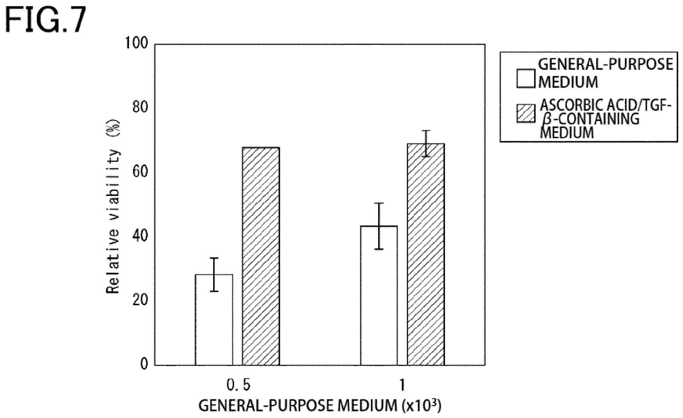

GFP expressed in the cancer cells in the three-dimensional cellular tissue was imaged using a microscope system (Operetta CLS, PerkinElmer) to include the entire inside of the cell culture insert. The obtained image was subjected to image analysis to calculate the percentage of cancer cells in the cell culture insert (confluency), and the confluency with addition of the CTL cells when the confluency with no addition of CTL cells (control) was taken as 100% was calculated as the relative cell viability (relative viability). Fig. 7 shows the results.

As shown in Fig. 7, in the three-dimensional cellular tissue cultured in a medium containing ascorbic acid and TGF-β, damage to cancer cells by CTL cells was suppressed. This shows that, in the three-dimensional cellular tissue cultured in a medium containing ascorbic acid and TGF-β, the cancer cells formed a stroma barrier to defend against attacks by CTL cells, resulting in the cancer cells having functional characteristics.

### [Experimental Example 4]

### (Production 3 of Three-Dimensional Cellular Tissues)

### <<Preparation of Stromal Cells>>

2.0×10⁶ cells of human neonatal dermal fibroblasts NHDF (product number "CC-2509", Lonza) and 3×10⁴ cells of human umbilical vein endothelial cells HUVEC (product number "C2517A", Lonza) were suspended in a 50 mM tris-HCl buffer solution (pH 7.4) containing 0.1 mg/mL heparin (product number "H3149-100KU", Sigma) and 0.1 mg/mL collagen (product number "ASC-1-100-100", Sigma).

Then, the cell suspension was centrifuged at room temperature at 1,000 × g (gravitational acceleration) for 2 minutes, and, after removing the supernatant, resuspended in 300 µL of general-purpose medium. Then, the obtained cell suspension was seeded inside a 24-well cell culture insert (product number "3470", Corning Incorporated) coated with 0.1 mg/mL fibronectin in advance, with 1 mL of general-purpose medium added to the outside.

Then, the cell culture insert was centrifuged at room temperature at 400 × g for 2 minutes, and then allowed to stand in a CO₂incubator (37°C, 5% CO₂) for 2 hours. Then, 1 mL of general-purpose medium was added to the outside the cell culture insert, and the cells were cultured in a CO₂ incubator (37°C, 5% CO₂) for 24 hours (the time at which this culture was started is referred to as the culture start time).

<<Addition of Ascorbic Acid and TGF-β>>

The medium inside and outside the above cell culture insert was removed, and 2.3 mL of general-purpose medium was added, using magnesium ascorbate phosphate (product number "013-12061", FUJIFELM Wako Pure Chemical Corporation) and TGF-β (product number "209-16544", FUJIFELM Wako Pure Chemical Corporation), with the ascorbic acid concentration being 0.1 mM and the TGF-β concentration being 5 ng/mL, and the cells were cultured in a CO₂ incubator (37°C, 5% CO₂) for 3 days. Then, the medium inside and outside the culture insert was removed, and 2.3 mL of general-purpose medium was added with the ascorbic acid concentration being 0.1 mM and the TGF-β concentration being 5 ng/mL, and the cells were cultured in a CO₂ incubator (37°C, 5% CO₂) for 3 days.

### <<Measurement of Thickness of Three-Dimensional Cellular Tissues>>

On day 4 and day 7 of culture, the obtained three-dimensional cellular tissue was removed from the cell culture insert, embedded in paraffin, and cut along a line passing through the center of gravity in a direction perpendicular to the top of the three-dimensional cellular tissue (top of the cell culture insert) to prepare a 6-mm thin slice. Then, the thin slice was stained with hematoxylin-eosin (HE), and the HE-stained thin slice was imaged using an optical microscope (MX51, Olympus Corporation) to measure a maximum thickness of the three-dimensional cellular tissue using ImageJ.

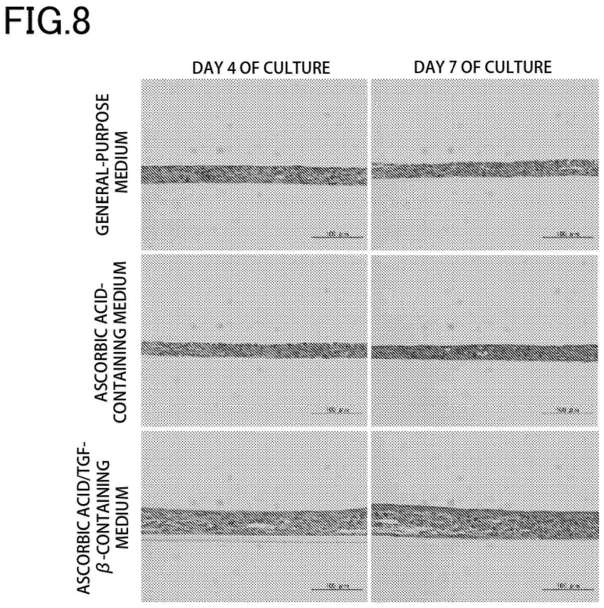

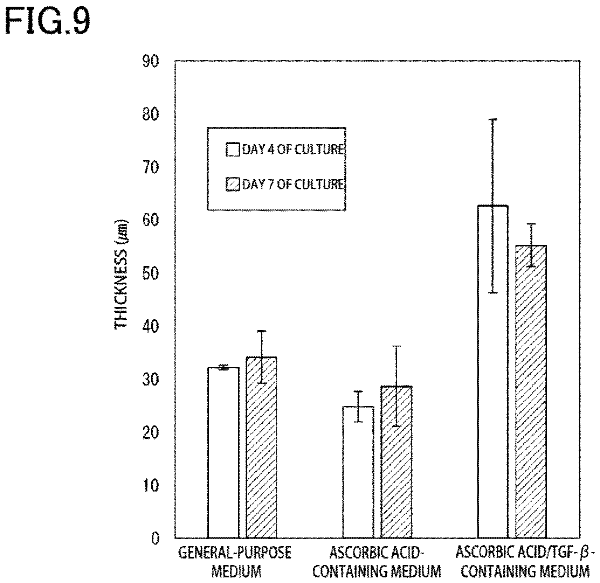

Fig. 8 shows the result of microscopic observation of the HE-stained three-dimensional cellular tissue, and Fig. 9 shows the result of measuring the thickness of the three-dimensional cellular tissue. As shown in Figs. 8 and 9, the thickness of the three-dimensional cellular tissue was maintained by adding ascorbic acid and TGF-β, and the thickness of the three-dimensional cellular tissue was found to be greater than 50 µm even on day 4 of culture (on day 3 after addition of ascorbic acid and TGF-β).

### [Industrial Applicability]

According to the present invention, a technique for producing three-dimensional cellular tissues can be provided that can suppress decrease in thickness of a three-dimensional cellular tissue even when cultured over time, maintain the thickness of the three-dimensional cellular tissue, and suppress decrease in collagen in the three-dimensional cellular tissue.

## Claims

1. A method of producing three-dimensional cellular tissues comprising the steps of:
mixing stromal cells with a cationic substance, an extracellular matrix component and a polyelectrolyte to obtain a mixture;
removing a liquid portion from the mixture to obtain a cell aggregate;
culturing the cell aggregate in a medium to obtain a three-dimensional cellular tissue; and
culturing the three-dimensional cellular tissue in a medium containing ascorbic acid and transforming growth factor-β (TGF-β), wherein
a thickness of the three-dimensional cellular tissue remains greater than 50 µm for at least 3 days after culturing the three-dimensional cellular tissue in the medium containing ascorbic acid and TGF-β.

2. A method of producing three-dimensional cellular tissues comprising the steps of:
mixing stromal cells with a cationic substance, an extracellular matrix component and a polyelectrolyte to obtain a first mixture;
removing a liquid portion from the mixture to obtain a first cell aggregate;
culturing the first cell aggregate in a medium to obtain a first three-dimensional cellular tissue;
disposing target cells on the first three-dimensional cellular tissue;
mixing stromal cells with a cationic substance, an extracellular matrix component and a polyelectrolyte to obtain a second mixture;
removing a liquid portion from the second mixture to obtain a second cell aggregate;
disposing the second cell aggregate to be in contact with the target cells;
and culturing the second cell aggregate in a medium containing ascorbic acid and transforming growth factor-β (TGF-β) to obtain a second three-dimensional cellular tissue, wherein
a total thickness of the first three-dimensional cellular tissue, the target cells and the second three-dimensional cellular tissue remains greater than 50 µm for at least 3 days after culturing the second three-dimensional cellular tissue in the medium containing ascorbic acid and TGF-β.

3. The production method according to claim 2, wherein the target cells are cancer cells.

4. The production method according to any one of claims 1 to 3, wherein
the extracellular matrix component is selected from the group consisting of collagen, laminin, fibronectin, vitronectin, elastin, tenascin, entactin, fibrin, proteoglycan, and combinations thereof.

5. The production method according to any one of claims 1 to 4, wherein
the polyelectrolyte is selected from the group consisting of glycosaminoglycan, dextran sulfate, rhamnan sulfate, fucoidan, carrageenan, polystyrene sulfonic acid, polyacrylamide-2-methylpropanesulfonic acid, polyacrylic acid, and combinations thereof.
